(19) Europäisches Patentamt
European Patent Office
Office européen des brevets

(11) EP 4 678 228 A1

(12) EUROPEAN PATENT APPLICATION

(43) Date of publication:
14.01.2026 Bulletin 2026/03

(51) International Patent Classification (IPC):
A61N 5/10 (2006.01)

(21) Application number: 25188130.6

(52) Cooperative Patent Classification (CPC):
A61N 5/1031

(22) Date of filing: 08.07.2025

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 10.07.2024 US 202418768510

(71) Applicant: Siemens Healthineers International AG
6312 Steinhausen (CH)

(72) Inventors:
• MAKKONEN, Jarmo
00520 Helsinki (FI)
• KORHONEN, Laura
02340 Espoo (FI)

(74) Representative: Mathisen & Macara LLP
Charta House
30-38 Church Street
Staines-upon-Thames TW18 4EP (GB)

(54) **RADIATION TREATMENT PLANNING METHODS AND APPARATUS**

(57) A control circuit 101 can be configured to generate 201 a virtual target volume 601, 701 as a function of a target volume 402 for a particular patient and to then generate 202 a virtual bolus 901 as a function of the virtual target volume. The control circuit can then optimize 203 a radiation treatment plan for that particular patient using the target volume and the virtual bolus to determine a corresponding radiation dose(s).

## 200

```
BY A CONTROL CIRCUIT
        │
        ▼
GENERATE A VIRTUAL TARGET VOLUME AS A FUNCTION OF
A TARGET VOLUME                                       — 201
        │
        ▼
GENERATE A VIRTUAL BOLUS AS A FUNCTION OF THE VIRTUAL
TARGET VOLUME                                         — 202
        │
        ▼
OPTIMIZE THE RADIATION TREATMENT PLAN USING THE TARGET
VOLUME AND THE VIRTUAL BOLUS TO DETERMINE RADIATION DOSE — 203
TO PROVIDE AN OPTIMIZED RADIATION TREATMENT PLAN
        │
        ▼
ADMINISTER THERAPEUTIC RADIATION TO A PARTICULAR PATIENT
USING THE OPTIMIZED RADIATION TREATMENT PLAN            — 204
```

FIG. 2

## Description

BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

There are potential sources for error when preparing a radiation treatment plan. Some examples include patient setup error, swelling of patient tissue, and patient movement (for example, due to breathing) during radiation delivery. These circumstances can be of concern when planning, for example, radiation treatment for a patient target volume that is close to the surface of the body (as is often the case when treating breast cancer).

Skin flash refers to an intentional extension of the radiation field beyond the patient's skin surface to ensure that the patient's treatment volume receives the full dose of radiation. The flash helps to ensures that a part of a patient treatment volume that is located within the skin, which may move or deform slightly during breathing or patient movement, is consistently covered by the therapeutic dose throughout the treatment.

SUMMARY

According to one aspect of the present invention, there is provided a method to facilitate optimizing a radiation treatment plan for a target volume for a particular patient, as defined in claim 1. Optional features are specified in the dependent claims.

According to another aspect of the present invention, there is provided an apparatus to facilitate optimizing a radiation treatment plan for a target volume for a particular patient, as defined in claim 10. Optional features are specified in the dependent claims.

BRIEF DESCRIPTION OF DRAWINGS

The above needs are at least partially met through provision of the radiation treatment planning method and apparatus described in the following detailed description, particularly when studied in conjunction with the drawings, wherein:

FIG. 1 comprises a block diagram as configured in accordance with various embodiments of these teachings;
FIG. 2 comprises a flow diagram as configured in accordance with various embodiments of these teachings;
FIG. 3 comprises a flow diagram as configured in accordance with various embodiments of these teachings;
FIG. 4 comprises a schematic representation as configured in accordance with various embodiments of these teachings;
FIG. 5 comprises a schematic representation as configured in accordance with various embodiments of these teachings;
FIG. 6 comprises a schematic representation as configured in accordance with various embodiments of these teachings;
FIG. 7 comprises a schematic representation as configured in accordance with various embodiments of the invention;
FIG. 8 comprises a schematic representation as configured in accordance with various embodiments of these teachings; and
FIG. 9 comprises a schematic representation as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

DETAILED DESCRIPTION

Generally speaking, pursuant to these various embodiments, a control circuit can be configured to generate a virtual target volume as a function of a target volume for a particular patient and to then generate a virtual bolus as a function of the virtual target volume. The control circuit can then optimize a radiation treatment plan for that particular patient using the target volume and the virtual bolus to determine a corresponding radiation dose.

By one approach, generating the virtual target volume can comprise generating a first intermediate virtual target volume that is at least partially disposed external to a body structure contour for the particular patient. By one approach, generating the aforementioned virtual target volume can include optionally providing a user opportunity to set a different user-provided extension value for each of six orthogonal directions (such as in a positive and a negative direction for each of the X axis, the Y axis, and the Z axis). Such extension values (or other extension values of choice) can be used to then dilate the target volume to provide a dilated target volume.

The control circuit can then subtract a body structure contour for the particular patient from the dilated target volume to produce a first intermediate virtual target volume. These teachings will then accommodate filling gaps between the target volume and the first intermediate virtual target volume to provide a second intermediate virtual target volume. By one approach, the virtual target volume can then be generated by separating the second intermediate virtual target volume from previously-generated target voxels that are separate from the target volume to provide the virtual target volume.

When generating the aforementioned virtual bolus, these teachings will accommodate determining a density for the virtual bolus.

These teachings are highly flexible in practice and will accommodate various application settings. By one approach, for example, the aforementioned virtual target volume can comprise a virtual skin flash target.

The optimized radiation treatment plan developed by these teachings can be used, for example, to administer therapeutic radiation to the particular patient.

So configured, these teachings can save planning time as compared, for example, to manual skin flash techniques. It will be appreciated that these teachings can facilitate varying the density of the aforementioned virtual bolus as a function of the radiation beam's direction. The latter capability, in turn, can lead to improved quality and efficacy of the resultant plan.

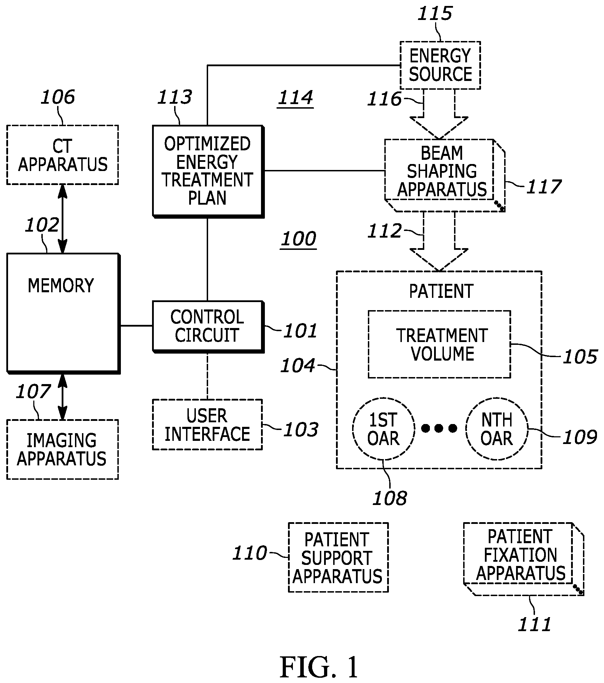

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patientrelated imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 (which is an apparatus that is comprised of a number of component apparatuses) that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

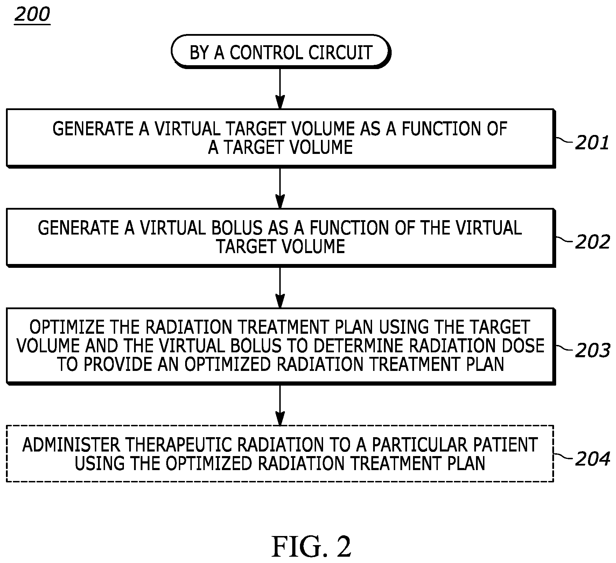

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for generating a virtual target volume as a function of a target volume in a particular patient (such as a tumor; for the sake of an illustrative example, this description will often presume the tumor to be located in the particular patient's breast, but it will be understood that these teachings are not limited to only that application setting.)

These teachings will accommodate any of a variety of ways to generate the virtual target volume.

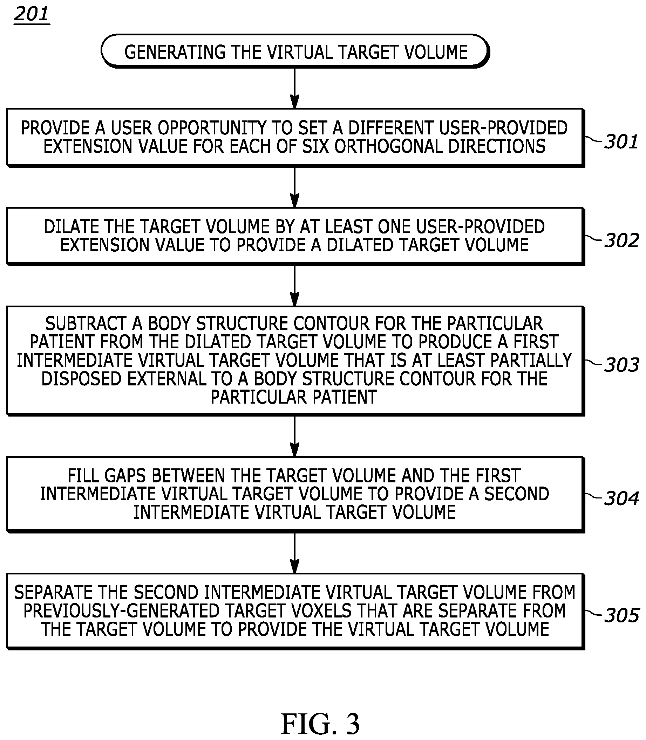

Referring momentarily to FIG. 3, various particular approaches in these regards will be described. Again, it will be understood that these teachings are not limited to only these described approaches.

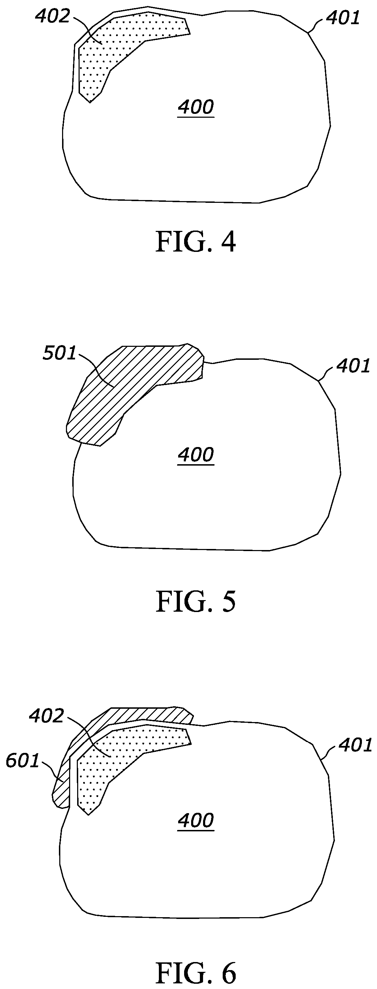

In addition, and for the sake of this illustrative example, FIG. 4 presents a representation of a patient volume 400 having an exterior surface 401 (i.e., skin) and a target volume 402 that is close to that exterior surface 401.

This process for generating the virtual target volume can make use of a user-provided extension value as described below.

By one approach, and as illustrated at block 301, this process can include providing a user opportunity (via, for example, the aforementioned user interface 103) to set a different (or the same) user-provided extension value for each of six orthogonal directions. Viewed generally, these six directions may comprise positive and negative directions in the X direction, positive and negative directions in the Y direction, and positive and negative directions in the Z direction. Viewed contextually for a relevant application setting, these six directions may comprise anterior, posterior, left, right, head, and feet-oriented directions as regards a patient.

At block 302, the control circuit 101 dilates the target volume 402 by at least one user-provided extension value (such as the aforementioned user-provided extension values for each of six orthogonal directions) to provide a dilated target volume 501 as shown in FIG. 5. In particular, the control circuit 101 dilates the target volume 402 by user-defined radii according to one or more of the aforementioned six main directions (namely the anterior, posterior, left, right, head, and feet directions in this illustrative example).

At block 303, the control circuit 101 then subtracts a body structure contour (defined, for example, by the particular patient's exterior surface 401) for the particular patient from the dilated target volume 501 to produce a first intermediate virtual target volume 601 as shown in FIG. 6. The latter, if present, serves to test whether the dilated target volume 501 indeed extends at any point beyond the patient's body. When such is not the case, this process can conclude in these regards (or, when applicable, the process can proceed in a similar manner for additional target volumes).

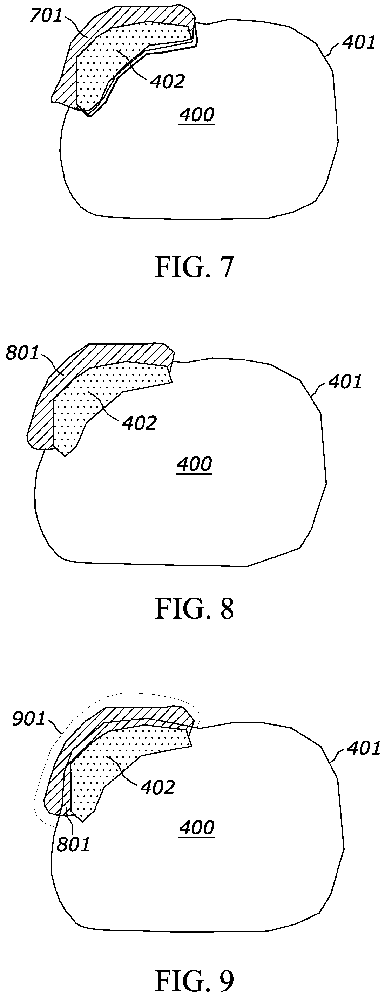

Presuming that the first intermediate virtual target volume 601 extends beyond the patient's body, at block 304 this process will optionally accommodate filling gaps between the target volume 402 and the first intermediate virtual target volume 601 to provide a second intermediate virtual target volume 701 as shown in FIG. 7. By one approach, the latter can comprise taking the union of the first intermediate virtual target volume 601 and the target volume 402 and then applying dilation by a fixed extension radius to all directions, followed by erosion by the same extension radius to three-dimensionally connect the structures, and then subtracting the target volume 402.

At block 305, this process can provide for then separating the second intermediate virtual target volume 701 from previously-generated target voxels that are separate from the target volume 402 to provide a resultant virtual target volume 801 as shown in FIG. 8. In effect, this activity can serve to clean up the resulting virtual target volume 801. By one approach, the latter can comprise doing morphological opening on the second intermediate virtual target volume 701 to remove any separate clusters of voxels that may remain from the previous operations. Removing these voxels can help to ensure that unnecessary radiation is not directed inside the patient's body.

This process can produce, for example, a virtual automatically-produced skin flash target that can be used in optimization in addition to the original structures.

Referring again to FIG. 2, and also to FIG. 9, at block 202 this process 200 can provide for generating a virtual bolus 901 as a function of the virtual target volume 801. (By one approach, a virtual bolus can be used to modify a computed tomography image of the patient such that the dose calculation produces dose to portions of the virtual target that may be located outside the original patient's body contour.) By one approach, this can comprise dilating the virtual target volume 801 by a predetermined bolus thickness (such as 3 mm, which may be a default value that is automatically applied or which may be a user-selected value, as desired), and then subtracting the patient's exterior surface 401 from the dilated structure.

By one approach, the virtual bolus 901 can have an automatically determined density (based, for example, on patient imagery). For example, a density value for the virtual bolus 901 can be automatically estimated from one or more computed tomography images by taking the Hounsfield Units (HU) values for the original target volume 402 and calculating a trimmed median. In lieu of the foregoing, or in combination therewith, the virtual bolus 901 can have a density that is assigned by a user (via, for example, the aforementioned user interface 103). The virtual bolus density can be added to one or more corresponding computed tomography images produced from other structures and density assignments as appropriate to the application setting.

At block 203, the control circuit 101 optimizes the radiation treatment plan using the target volume 402 and the virtual bolus 901 to determine at least one corresponding radiation dose to thereby provide an optimized radiation treatment plan 113. In particular, the virtual bolus 901 can be referenced during optimization to produce dose to the virtual target volume 801. For many applications settings, optimization can comprise optimizing the radiation treatment plan using the original target volume, the virtual target volume, the virtual bolus, and the organs-at-risk to provide an optimized radiation treatment plan (such that both the original target volume and the new virtual target volume(s) are used during optimization). In particular, all of the organ-at-risk structures for which the user had defined objectives can be used when optimizing the radiation treatment plan. By one approach, during dose calculation, the radiological density for each beamlet can be further scaled by a factor that is a function of the angle between the beamlet and the average of surface normal directions over k (>0) nearest points on the patient's body where the beamlet hits.

By one approach, during the optimization process both the original and the virtual target volume are optimization targets. In these regards, the optimization objectives for the original target volume can be copied to the virtual target.

At optional block 204, the control circuit 101 can then administer therapeutic radiation 112 to the particular patient 104 using the optimized radiation treatment plan 113.

Further details that comport with these teachings will now be presented. It will be understood that the specific details of these examples are intended to serve an illustrative purpose and are not intended to suggest any particular limitations with respect to these teachings.

Dose estimation during optimization can use electron density (derived, for example, from computed tomography imagery) for inhomogeneity correction. The virtual bolus density can be added to the computed tomography image per dose calculation field (for example, the treatment fields for intensity-modulated radiation therapy or a subset of control points along each arc field for volumetric modulated arc therapy), so that the density in each voxel $\rho$ is a function of the angle $\theta$ between the beam and the average of surface normal directions over k (>0) nearest points on body to the voxel:

$$\rho = f(\theta)$$

Possible forms of the function can be, but are not limited to:

$$x = \frac{(\cos\theta_0 - \|\cos(\theta)\|)}{\cos\theta_0}$$

$$\rho = \rho_0 \begin{cases} 0, & x < 0 \\ x, & x \geq 0 \end{cases}$$

where $\cos\overline{\theta}_0$ is the cosine of a (user-defined) reference angle value $\overline{\theta}_0$ and $\rho_0$ is a (user-defined) default electron density value for the virtual bolus.

The above function can ramp up from zero at the limit angle value to $p_0$ at a tangential position (with the body surface at a right angle to the beam direction):

$$\rho = \rho_0 H(\cos\theta_0 - \|\cos(\theta)\|)$$

$\rho = \rho_0 H(\cos\theta_0 - \|\cos(\theta)\|)$ where H is the Heaviside step function (zero for negative argument and one for positive argument). This function can yield a sharp edge in the density, without any ramp-up.

These teachings will accommodate automatically determining the virtual bolus HU value as a trimmed median of the HU values for the original target in the computed tomography image. As used herein, the expression "trimmed median" means considering only HU values within a predetermined range for suitable values that represent organ tissue, and therefore removing values that correspond to air and/or extremely dense volumes.

It will be appreciated that, by creating the aforementioned virtual bolus based on a virtual target instead of an original target, these teachings tend to avoid creating an excessively large virtual bolus. By one approach, when scaling the virtual bolus density with a scaling factor that depends on the direction of the beamlet with respect to the virtual bolus, it is not necessary to create a copy of the computed tomography image per each calculation direction. Instead, the radiological path length for each beamlet can be scaled by this factor during dose calculations.

It will also be appreciated that these teachings allow for the possibility of using intermediate dose calculation during optimization together with automatic skin flash accommodation per the foregoing. Since the intermediate dose calculation is done during optimization, this calculation can also use the virtual structures created for the skin flash.

Further aspects of the invention are provided by the subject matter of the following clauses:

Clause 1. A method to facilitate optimizing a radiation treatment plan for a target volume for a particular patient, the method comprising: by a control circuit: generating a virtual target volume as a function of the target volume; generating a virtual bolus as a function of the virtual target volume; and optimizing the radiation treatment plan using the target volume, the virtual target volume, and the virtual bolus to determine radiation dose to provide an optimized radiation treatment plan.

Clause 2. The method of clause 1 wherein generating a virtual bolus includes determining a density for the virtual bolus.

Clause 3. The method of either of clause 1 or 2 wherein generating the virtual target volume comprises generating a first intermediate virtual target volume that is at least partially disposed external to a body structure contour for the particular patient.

Clause 4. The method of clause 3 wherein generating the first intermediate virtual target volume includes dilating the target volume by at least one user-provided extension value to provide a dilated target volume.

Clause 5. The method of clause 4 further comprising: providing a user opportunity to set a different user-provided extension value for each of six orthogonal directions.

Clause 6. The method of clause 4 further comprising: subtracting a body structure contour for the particular patient from the dilated target volume to produce the first intermediate virtual target volume.

Clause 7. The method of clause 6 further comprising: filling gaps between the target volume and the first intermediate virtual target volume to provide a second intermediate virtual target volume.

Clause 8. The method of clause 7 further comprising: separating the second intermediate virtual target volume from previously-generated target voxels that are separate from the target volume to provide the virtual target volume.

Clause 9. The method of any of clauses 1 through 8 wherein the virtual target volume comprises a virtual skin flash target.

Clause 10. The method of any of clauses 1 through 9 comprising: administering therapeutic radiation to the particular patient using the optimized radiation treatment plan.

Clause 11. An apparatus to facilitate optimizing a radiation treatment plan for a target volume for a particular patient, the apparatus comprising: a control circuit configured to: generate a virtual target volume as a function of the target volume; generate a virtual bolus as a function of the virtual target volume; and optimize the radiation treatment plan using the target volume and the virtual bolus to determine radiation dose to provide an optimized radiation treatment plan.

Clause 12. The apparatus of clause 11 wherein the control circuit is further configured to generate the virtual bolus by determining a density for the virtual bolus.

Clause 13. The apparatus of either of clause 11 or 12 wherein the control circuit is configured to generate the virtual target volume by generating a first intermediate virtual target volume that is at least partially disposed external to a body structure contour for the particular patient.

Clause 14. The apparatus of clause 13 wherein the control circuit is configured to generate the first intermediate virtual target volume by dilating the target volume by at least one user-provided extension value to provide a dilated target volume.

Clause 15. The apparatus of clause 14 wherein the control circuit is further configured to: provide a user opportunity to set a different user-provided extension value for each of six orthogonal directions.

Clause 16. The apparatus of clause 14 wherein the control circuit is further configured to: subtract a body structure contour for the particular patient from the dilated target volume to produce the first intermediate virtual target volume.

Clause 17. The apparatus of clause 16 wherein the control circuit is further configured to: fill gaps between the target volume and the first intermediate virtual target volume to provide a second intermediate virtual target volume.

Clause 18. The apparatus of clause 17 wherein the control circuit is further configured to: separate the second intermediate virtual target volume from previously-generated target voxels that are separate from the target volume to provide the virtual target volume.

Clause 19. The apparatus of any of clauses 11 through 18 wherein the virtual target volume comprises a virtual skin flash target.

Clause 20. The apparatus of any of clauses 11 through 19 wherein the control circuit is further configured to: administer therapeutic radiation to the particular patient using the optimized radiation treatment plan.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

**Claims**

1.  A method to facilitate optimizing a radiation treatment plan for a target volume for a particular patient, the method comprising:
    by a control circuit:

    generating a virtual target volume as a function of the target volume;
    generating a virtual bolus as a function of the virtual target volume; and
    optimizing the radiation treatment plan using the target volume and the virtual bolus to determine radiation dose to provide an optimized radiation treatment plan.

2.  The method of claim 1 wherein generating a virtual bolus includes determining a density for the virtual bolus.

3.  The method of claim 1 or 2 wherein generating the virtual target volume comprises generating a first intermediate virtual target volume that is at least partially disposed external to a body structure contour for the particular patient.

4.  The method of claim 3 wherein generating the first intermediate virtual target volume includes dilating the target volume by at least one user-provided extension value to provide a dilated target volume.

5.  The method of claim 4 further comprising:
    providing a user opportunity to set a different user-provided extension value for each of six orthogonal directions.

6. The method of claim 4 or 5 further comprising: subtracting a body structure contour for the particular patient from the dilated target volume to produce the first intermediate virtual target volume.

7. The method of claim 6 further comprising: filling gaps between the target volume and the first intermediate virtual target volume to provide a second intermediate virtual target volume.

8. The method of claim 7 further comprising: separating the second intermediate virtual target volume from previously-generated target voxels that are separate from the target volume to provide the virtual target volume.

9. The method of any one of claims 1 to 8 wherein the virtual target volume comprises a virtual skin flash target.

10. An apparatus to facilitate optimizing a radiation treatment plan for a target volume for a particular patient, the apparatus comprising: a control circuit configured to:

   generate a virtual target volume as a function of the target volume;
   generate a virtual bolus as a function of the virtual target volume; and
   optimize the radiation treatment plan using the target volume and the virtual bolus to determine radiation dose to provide an optimized radiation treatment plan.

11. The apparatus of claim 10 wherein the control circuit is further configured to generate the virtual bolus by determining a density for the virtual bolus.

12. The apparatus of claim 10 or 11 wherein the control circuit is configured to perform the method of any one of claims 3 to 8.

13. The apparatus of claim 10, 11 or 12 wherein the virtual target volume comprises a virtual skin flash target.

14. The apparatus of claim 10, 11, 12 or 13 wherein the control circuit is further configured to: administer therapeutic radiation to the particular patient using the optimized radiation treatment plan.

FIG. 1

_200_

```
( BY A CONTROL CIRCUIT )
            │
            ▼
┌──────────────────────────────────────────────────┐
│ GENERATE A VIRTUAL TARGET VOLUME AS A FUNCTION OF  │ ─ 201
│               A TARGET VOLUME                      │
└──────────────────────────────────────────────────┘
            │
            ▼
┌──────────────────────────────────────────────────┐
│ GENERATE A VIRTUAL BOLUS AS A FUNCTION OF THE VIRTUAL │ ─ 202
│               TARGET VOLUME                        │
└──────────────────────────────────────────────────┘
            │
            ▼
┌──────────────────────────────────────────────────┐
│ OPTIMIZE THE RADIATION TREATMENT PLAN USING THE TARGET │
│ VOLUME AND THE VIRTUAL BOLUS TO DETERMINE RADIATION DOSE │ ─ 203
│ TO PROVIDE AN OPTIMIZED RADIATION TREATMENT PLAN   │
└──────────────────────────────────────────────────┘
            │
            ▼
┌──────────────────────────────────────────────────┐
│ ADMINISTER THERAPEUTIC RADIATION TO A PARTICULAR PATIENT │ ─ 204
│     USING THE OPTIMIZED RADIATION TREATMENT PLAN   │
└──────────────────────────────────────────────────┘
```

FIG. 2

_201_

```
( GENERATING THE VIRTUAL TARGET VOLUME )
```

PROVIDE A USER OPPORTUNITY TO SET A DIFFERENT USER-PROVIDED EXTENSION VALUE FOR EACH OF SIX ORTHOGONAL DIRECTIONS ~_301_

DILATE THE TARGET VOLUME BY AT LEAST ONE USER-PROVIDED EXTENSION VALUE TO PROVIDE A DILATED TARGET VOLUME ~_302_

SUBTRACT A BODY STRUCTURE CONTOUR FOR THE PARTICULAR PATIENT FROM THE DILATED TARGET VOLUME TO PRODUCE A FIRST INTERMEDIATE VIRTUAL TARGET VOLUME THAT IS AT LEAST PARTIALLY DISPOSED EXTERNAL TO A BODY STRUCTURE CONTOUR FOR THE PARTICULAR PATIENT ~_303_

FILL GAPS BETWEEN THE TARGET VOLUME AND THE FIRST INTERMEDIATE VIRTUAL TARGET VOLUME TO PROVIDE A SECOND INTERMEDIATE VIRTUAL TARGET VOLUME ~_304_

SEPARATE THE SECOND INTERMEDIATE VIRTUAL TARGET VOLUME FROM PREVIOUSLY-GENERATED TARGET VOXELS THAT ARE SEPARATE FROM THE TARGET VOLUME TO PROVIDE THE VIRTUAL TARGET VOLUME ~_305_

FIG. 3

FIG. 4

FIG. 5

FIG. 6

FIG. 7

FIG. 8

FIG. 9

Europäisches Patentamt
European Patent Office
Office européen des brevets

**EUROPEAN SEARCH REPORT**

Application Number

**EP 25 18 8130**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/100360 A1 (KUUSELA ESA [FI]) 28 March 2024 (2024-03-28) | 1-7,9-13 | INV. A61N5/10 |
| A | * figures 1, 4, 5 * <br> * paragraphs [0001], [0021] * <br> * paragraphs [0043] - [0046] * <br> ----- | 8 | |
| X | WO 2023/088897 A1 (SIEMENS HEALTHINEERS INT AG [CH]) 25 May 2023 (2023-05-25) | 1,2, 9-11,13 | |
| A | * paragraphs [0004] - [0008] * <br> * paragraph [0039] * <br> * paragraphs [0067] - [0072] * <br> ----- | 3-8,12, 14 | |
| X | TYRAN MARGUERITE ET AL: "Safety and benefit of using a virtual bolus during treatment planning for breast cancer treated with arc therapy", JOURNAL OF APPLIED CLINICAL MEDICAL PHYSICS, vol. 19, no. 5, 30 June 2018 (2018-06-30), pages 463-472, XP093326375, US ISSN: 1526-9914, DOI: 10.1002/acm2.12398 | 1-7,9-12 | |
| | | | **TECHNICAL FIELDS SEARCHED (IPC)** |
| A | * figures 1, 3 * <br> * Sections: 2 and 4 * <br> ----- | 8 | A61N |

The present search report has been drawn up for all claims

| Place of search | Date of completion of the search | Examiner |
|---|---|---|
| Munich | 21 October 2025 | Seiferle, Benedict |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 8130

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2025

| Patent document cited in search report | | Publication date | Patent family member(s) | | Publication date |
|---|---|---|---|---|---|
| US 2024100360 | A1 | 28-03-2024 | CN | 119947792 A | 06-05-2025 |
| | | | EP | 4593949 A1 | 06-08-2025 |
| | | | US | 2024100360 A1 | 28-03-2024 |
| | | | WO | 2024068249 A1 | 04-04-2024 |
| WO 2023088897 | A1 | 25-05-2023 | CN | 118265555 A | 28-06-2024 |
| | | | EP | 4433158 A1 | 25-09-2024 |
| | | | US | 12119102 B1 | 15-10-2024 |
| | | | WO | 2023088897 A1 | 25-05-2023 |

EPO FORM P0459

For more details about this annex : see Official Journal of the European Patent Office, No. 12/82